# EUROPEAN PATENT APPLICATION

(11) **EP 2 143 739 A1**
(43) Date of publication of application: **13.01.2010**
(21) Application number: 08740816.7
(22) Date of filing: 24.04.2008
(51) Int. Cl.: C08F 20/36, C09D 7/12, C09D 175/04, C09J 11/06, C09J 175/04, C08G 18/81

(54) **URETHANE COMPOUND, CURABLE COMPOSITION CONTAINING THE SAME, AND CURED PRODUCT OF THE COMPOSITION**

(30) Priority: 27.04.2007 JP 2007118929; 28.11.2007 JP 2007307187
(71) Applicant: Showa Denko K.K., Tokyo 105-8518 (JP)
(72) Inventor: URAKAWA, Yoshifumi, Tokyo 105-8518 (JP); ISHII, Nobuaki, Tokyo 105-8518 (JP); MITARAI, Nobuyuki, Tokyo 105-8518 (JP); HATTORI, Yotaro, Tokyo 105-8518 (JP); OI, Hiroko, Tokyo 105-8518 (JP); MUROFUSHI, Katsumi, Tokyo 105-8518 (JP)
(74) Representative: Strehl Schübel-Hopf & Partner
(86) International application number: PCT/JP2008/057889
(87) International publication number: WO 2008/136334

(57) **Abstract**

It is an object of the present invention to provide a urethane compound capable of providing a cured product which is excellent in surface hardness, scratch resistance, flexibility and bending property, has reduced curling property and also has good transparency, a curable composition containing the same and a cured product thereof. The curable composition of the present invention is **characterized by** containing a specific urethane compound having an isocyanurate ring skeleton and a polymerization initiator.

## Description

### TECHNICAL FIELD

The present invention relates to a urethane compound containing an isocyanurate ring skeleton, a curable composition containing the same and a cured product thereof.

### BACKGROUND ART

Resin cured products having an isocyanurate ring as a main skeleton are excellent in heat resistance property, has good surface smoothness and canmaintain light transmission property even in thermal storage. Therefore, they are used as coating materials for purpose of decoration or protection and are utilized for displays of liquid crystal televisions, personal computers and cell phones.

For the purpose of protection, the resin cured products need a certain surface hardness, and it is known that introducing a polymerizable functional group (e.g., olefin, hydroxyl group or the like) into a compound constituting the resin cured products makes it possible to enhance the surface hardness thereof.

Further, in recent years, as protective coating materials to prevent scratches or stains of various base material surfaces, adhesives or sealing materials for various base materials, curable compositions capable of forming cured films excellent in hardness, flexibility, scratch resistance, abrasion resistance, low curling property (this means small warpage of cured films formed by curing of the curable compositions), adhesion and transparency on surfaces of various base materials have been required.

Moreover, curable compositions capable of forming cured films excellent in hardness, flexibility, scratch resistance, abrasion resistance, low curling property, high refractive index, adhesion and transparency have been required in use applications of film type liquid crystal elements, touch panels and antireflection films of plastic optical parts or the like.

However, when trifunctional or higher polyfunctional acrylate having an isocyanurate ring as a main skeleton is used, its curing rate is high, but a problem of poor flexibility of a cured product has been found, and the coating film tends to suffer from occurrence of cracking, peeling or curling.

In order to prevent cracking of the coating film, improvements, such as decrease of a functional group concentration of the polyfunctional acrylate that is a main component of the coating film and increase of a molecular weight, have been studied. They, however, have a bad influence such as lowering surface hardness or scratch resistance of the cured product, and it is sometimes difficult to obtain a favorable hard coat film.

Furthermore, various compositions have been proposed, but in the existing circumstances, any curable composition capable of providing a cured film having properties of high hardness and excellent flexibility has not been obtained yet.

Techniques having been intended to overcome these problems are described in the following Patent Gazette etc.

In Japanese Patent Laid-Open Publication No. 2002-67238 (patent document 1) and Japanese Patent Laid-Open Publication No. 2002-69333 (patent document 2), a composition comprising polyfunctional urethane acrylate, colloidal silica and a compound having a tetrahydrofurfuryl group and a (meth)acrylate in a molecule is disclosed. In the composition, however, the colloidal silica has no functional group, so that sufficient surface hardness cannot be obtained, and in addition, there is a problem of insufficient transparency of a cured film.

In Japanese Patent Laid-Open Publication No. H05-320289 (patent document 3), a photocurable composition containing tris[2-(acryloyloxy)ethyl]isocyanurate that is an acrylate having an isocyanurate ring structure and colloidal silica is disclosed. However, reduction of curling property is not described at all, and there is a concern that warpage of a coating film due to curing shrinkage takes place.

In Japanese Patent Laid-Open Publication No. 2006-168238 (patent document 4), a curable composition using trimethylolpropane tri(meth)acrylate is disclosed. However, transmittance, curling property and curability are not described at all, and there is a concern that lowering of transparency or warpage of a coating film due to curing shrinkage takes place.

In Japanese Patent Laid-Open Publication No. H08-259644 (patent document 5), a curable composition using urethane acrylate composed of bisphenol type polyol and an ethylenic unsaturated monomer is disclosed. In this document, improvements in scratch resistance and flexibility have been studied, but even in this technique, there is yet a room for improvement in the scratch resistance.

In Japanese Patent Laid-Open Publication No. 2005-113033 (patent document 6), a curable composition containing low-molecular weight urethane (meth)acrylate is disclosed, and improvements in scratch resistance and flexibility have been made. Even in a method using this technique, however, there is yet a room for improvement in the scratch resistance.
Patent document 1: Japanese Patent Laid-Open Publication No. 2002-67238
Patent document 2: Japanese Patent Laid-Open Publication No. 2002-69333
Patent document 3: Japanese Patent Laid-Open Publication No. H05-320289
Patent document 4: Japanese Patent Laid-Open Publication No. 2006-168238
Patent document 5: Japanese Patent Laid-Open Publication No. H08-259644
Patent document 6: Japanese Patent Laid-Open Publication No. 2005-113033

### DISCLOSURE OF THE INVENTION

### PROBLEM TO BE SOLVED BY THE INVENTION

It is an object of the present invention to provide a urethane compound capable of providing a cured product which is excellent in surface hardness and scratch resistance, has reduced curling property and also has good transparency, a curable composition containing the same and a cured product thereof.

It is another obj ect of the present invention to provide a curable composition capable of providing a cured film which has tenacious strength typified by bending property or elongation property, and flexibility that are compatible with each other with maintaining the above-mentioned respective properties of surface hardness, scratch resistance, curling property and transparency, and a cured product thereof.

### MEANS TO SOLVE THE PROBLEM

In order to solve the above problems, the present inventors have earnestly studied, and as a result, they have found that the above problems can be solved by a urethane compound having a specific structure and a curable composition containing the same.

That is to say, the present invention is summarized as follows.

[1] A urethane compound represented by the following general formula (1):

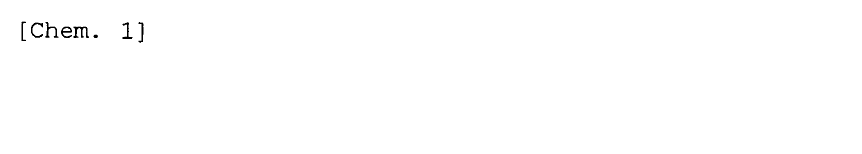

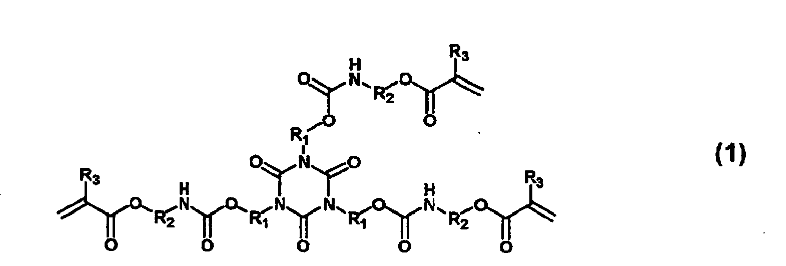

wherein R₁ is a straight-chain or branched divalent aliphatic group of 1 to 12 carbon atoms, a divalent organic group of 3 to 12 carbon atoms having an alicyclic group, a divalent organic group having an aromatic ring or [-(CH₂)ₐ-O-(CH₂)_{b}-]_{c} (a and b are each independently an integer of 1 to 10, and c is an integer of 1 to 5),
R₂ is a straight-chain or branched divalent aliphatic group of 1 to 10 carbon atoms, a divalent organic group of 3 to 10 carbon atoms having an alicyclic group, a divalent organic group having an aromatic ring or [-(CH₂)_{d}-O-(CH₂)ₑ-]_{f} (d and e are each independently an integer of 1 to 10, and f is an integer of 1 to 5), and
R₃ is a hydrogen atom or a methyl group.

[2] The urethane compound as stated in [1], wherein in the general formula (1), R₁ is a divalent aliphatic group of 1 to 6 carbon atoms,
R₂ is a divalent saturated aliphatic group of 1 to 6 carbon atoms, a divalent organic group having an benzene ring or [-(CH₂)_{d}-O-(CH₂)ₑ-]_{f} (d and e are each independently an integer of 1 to 5, and f is an integer of 1 to 3), and
R₃ is a hydrogen atom.

[3] A curable composition containing the urethane compound (A) as stated in [1] and a polymerization initiator (B).

[4] The curable composition as stated in [3], further containing a urethane oligomer (C).

[5] The curable composition as stated in [3] or [4], further containing a reactive monomer (D).

[6] The curable composition as stated in any one of [3] to [5], further containing a thiol compound (E).

[7] The curable composition as stated in [6], wherein the thiol compound (E) has two or more structures represented by the following formula (2):

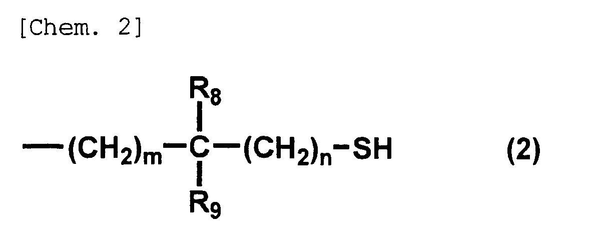

wherein R₈ and R₉ are each independently a hydrogen atom, an alkyl group of 1 to 10 carbon atoms or an aryl group, m is an integer of 0 to 2, n is 0 or 1, and R₈ and R₉ are not hydrogen atoms at the same time.
[8] The curable composition as stated in any one of [3] to [7], further containing a urethane compound (F) represented by the following general formula (3):

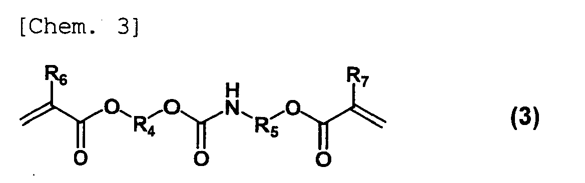

wherein R₄ is a straight-chain or branched divalent aliphatic group of 1 to 12 carbon atoms, a divalent organic group of 3 to 12 carbon atoms having an alicyclic group, a divalent organic group having an aromatic ring or [-(CH₂)_{g}-O-(CH₂)ₕ-]ᵢ (g and h are each independently an integer of 1 to 10, and i is an integer of 1 to 5),
R₅ is a straight-chain or branched divalent aliphatic group of 1 to 10 carbon atoms, a divalent organic group of 3 to 10 carbon atoms having an alicyclic group, a divalent organic group having an aromatic ring or [-(CH₂)ₚ-O-(CH₂)_{q}-]ᵣ (p and q are each independently an integer of 1 to 10, and r is an integer of 1 to 5), and
R₆ and R₇ are each independently a hydrogen atom or a methyl group.

[9] A paint comprising the curable composition as stated in any one of [3] to [8].

[10] An adhesive comprising the curable composition as stated in any one of [3] to [8].

[11] A cured product obtained by curing the curable composition as stated in any one of [3] to [8].

[12] A coating material obtained by curing the curable composition as stated in any one of [3] to [8].

[13] An optical film obtained by curing the curable composition as stated in any one of [3] to [8].

### EFFECT OF THE INVENTION

According to the present invention, provided are a urethane compound capable of forming a cured product which is excellent in surface hardness and scratch resistance, has reduced curling property in the case of a cured film, has good transparency and has tenacious strength typified by bending property or elongation property, and flexibility that are compatible with each other, a curable composition containing the urethane compound and a cured product obtained by curing the curable composition.

### BRIEF DESCRIPTION OF THE DRAWINGS

Fig. 1 shows a ¹H-NMR chart of a urethane compound (A-1) synthesized in Synthesis Example 1.
Fig. 2 shows a ¹H-NMR chart of a urethane compound (A-2) synthesized in Synthesis Example 2.

### BEST MODE FOR CARRYING OUT THE INVENTION

The embodiment of the present invention is described in detail hereinafter.

### [(A) Urethane compound]

The urethane compound of the present invention (also referred to as a "urethane compound (A)" hereinafter) is represented by the following general formula (1), and contains 3 or more polymerizable unsaturated bonds in a molecule.

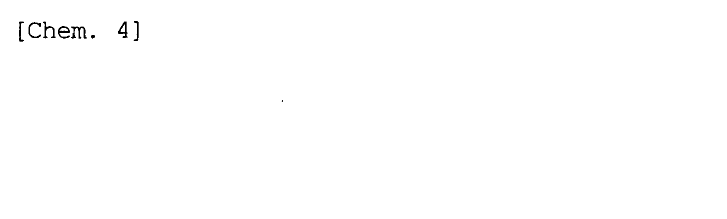

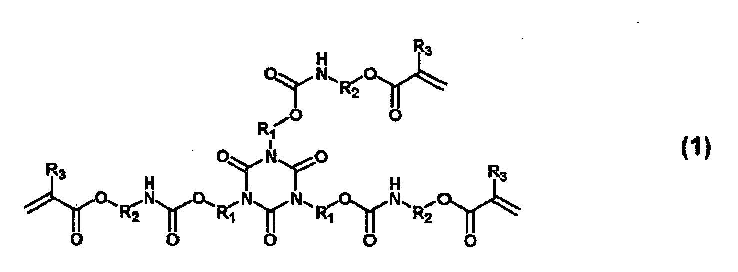

In the general formula (1), R₁ is a straight-chain or branched divalent aliphatic group of 1 to 12 carbon atoms, a divalent organic group of 3 to 12 carbon atoms having an alicyclic group, a divalent organic group having an aromatic ring or [-(CH₂)ₐ-O-(CH₂)_{b}-]_{c} (a and b are each independently an integer of 1 to 10, and c is an integer of 1 to 5 ).

From the viewpoint of a balance between hardness and transparency of a cured product obtained by curing the later-described curable composition of the present invention (also referred to as a "cured product" simply hereinafter), R₁ is preferably a divalent aliphatic group of 1 to 6 carbon atoms or [- (CH₂)ₐ-O-(CH₂)_{b}-]_{c} (a and b are each independently an integer of 1 to 5, and c is an integer of 1 to 3), more preferably a divalent aliphatic group of 2 to 4 carbon atoms or [-(CH₂)ₐ-O-(CH₂)_{b}-]_{c} (a and b are each independently an integer of 2 to 4, and c is 1 or 2), still more preferably a divalent aliphatic group of 2 carbon atoms or -(CH₂)₂-O-(CH₂)₂-.

In the general formula (1), R₂ is a straight-chain or branched divalent aliphatic group of 1 to 10 carbon atoms, a divalent organic group of 3 to 10 carbon atoms having an alicyclic group, a divalent organic group having an aromatic ring or [-(CH₂)_{d}-O-(CH₂)ₑ-]_{f} (d and e are each independently an integer of 1 to 10, and f is an integer of 1 to 5).

From the viewpoint of curability of the later-described curable composition of the present invention and the viewpoint of a balance between hardness and transparency of a cured product obtained by curing the curable composition, R₂ is preferably a divalent saturated aliphatic group of 1 to 6 carbon atoms, a divalent organic group having a benzene ring or [-(CH₂)_{d}-O-(CH₂)ₑ-]_{f} (d and e are each independently an integer of 1 to 5, and f is an integer of 1 to 3), more preferably a divalent saturated aliphatic group of 2 to 6 carbon atoms, a divalent organic group having a benzene ring or [-(CH₂)_{d}-O-(CH₂)ₑ-]_{f} (d and e are each independently an integer of 2 to 4, and f is 1 or 2), still more preferably a divalent saturated aliphatic group of 2 or 3 carbon atoms or -(CH₂)₂-O-(CH₂)₂-.

In the general formula (1), R₃ is a hydrogen atom or a methyl group, and from the viewpoint of curability that means being cured in a small light-exposed dose, R₃ is preferably a hydrogen atom.

The urethane compound (A) for use in the present invention can be synthesized by bringing an isocyanurate compound having a hydroxyl group, which is represented by the following general formula (4) (also referred to as a "compound (1)" hereinafter), into contact with a polymerizable unsaturated group-containing isocyanate compound represented by the following general formula (5) (also referred to as a "compound (2)" hereinafter).

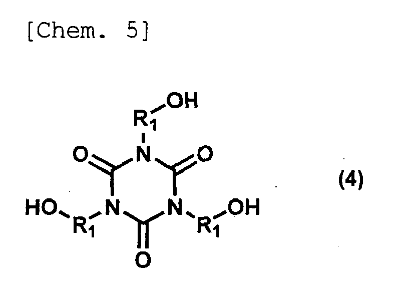

In the formula (4), R₁ is the same as that previously described.

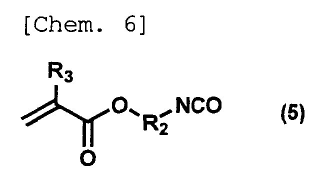

In the formula (5), R₂ and R₃ are the same as those previously described.

Formation of the urethane compound (A) can be confirmed by, for example, ¹H-NMR.

Examples of the compounds (1) include
tris(2-hydroxyethyl)isocyanurate, and its derivatives such as tris(2-hydroxypropyl)isocyanurate and
tris(2-hydroxybutyl)isocyanurate. These compounds can be used singly or as a mixture. Preferable is tris(2-hydroxyethyl)isocyanurate.

The compound (2) represented by the general formula (5) is the same as R₂ in the compound represented by the general formula (1). Examples of OCN-R₂- include the following.

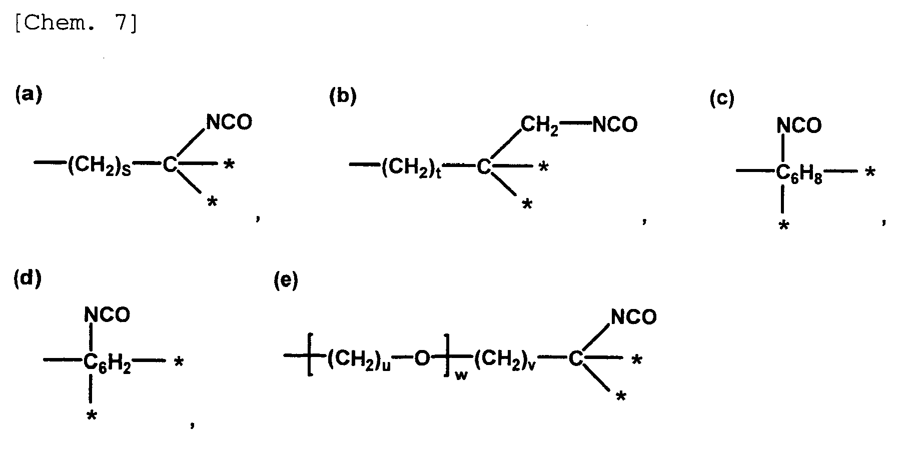

In the above formulas, * is a structure containing an isocyanate group, hydrogen, a (meth) acrylic group, a hydrocarbon group or an aromatic ring. Each * may be the same or different. (a) is a structure containing a divalent saturated aliphatic group having a straight-chain, branched or ring structure or containing an aromatic ring, and s is an integer satisfying the condition that the number of carbon atoms of the moiety excluding the isocyanate group is not less than 1 but not more than 10. (b) is a structure containing a divalent saturated aliphatic group having a straight-chain, branched or ring structure or containing an aromatic ring, and t is an integer satisfying the condition that the number of carbon atoms of the moiety excluding the isocyanate group is not less than 1 but not more than 10. (c) is a structure having a cyclohexane ring as a main skeleton, and the number of carbon atoms of the moiety excluding the isocyanate group is not less than 6 but not more than 10. (d) is a structure having a benzene ring as a main skeleton, and the number of carbon atoms of the moiety excluding the isocyanate group is not less than 6 but not more than 10. (e) is a structure having an ether skeleton, and u, v and w are each an integer satisfying the condition that the number of carbon atoms of the moiety excluding the isocyanate group is not less than 2 but not more than 100.

In the general formula (5), R₃ is a hydrogen atom or a methyl group, preferably a hydrogen atom.

Preferred examples of the compounds (2) are given below. Examples of those having the structure of (a) include
2-(meth)acryloyloxyethyl isocyanate, 3-(meth)acryloyloxypropyl isocyanate, 4-(meth)acryloyloxybutyl isocyanate,
5-(meth)acryloylpentyl isocyanate, 6-(meth)acryloyloxyhexyl isocyanate, 2-(meth)acryloyloxypropyl isocyanate and 1,1-bis(acryloyloxymethyl)ethyl isocyanate.
Examples of those having the structure of (b) include 3-isocyanato-2-methylpropyl (meth)acrylate.
Examples of those having the structure of (c) include 3-isocyanatocyclohexyl (meth)acrylate and 4-isocyanatocyclohexyl (meth)acrylate.
Examples of those having the structure of (d) include 3-isocyanatophenyl (meth)acrylate, 4-isocyanatophenyl (meth)acrylate and3,5-diisocyanato-2-methylphenyl (meth)acrylate.
Examples of those having the structure of (e) include 2-(isocyanatoethyloxy)ethyl (meth)acrylate.

In the case where R₃ is a hydrogen atom, that is, in the case where the compound (2) has an acrylate structure, curability of a curing composition containing the resulting urethane compound tends to be better than in the case where R₃ is a methyl group, that is, in the case where the compound (2) has a methacrylate structure.

In the reaction of the compound (1) with the compound (2), the molar ratio between them is in the range of 1:3 to 1:3.5 (compound (1):compound (2)).

In the reaction of the compound (1) with the compound (2), it is preferable to use a urethanization catalyst. By the use of the urethanization catalyst, the reaction can be remarkably accelerated. Specific examples of the urethanization catalysts include dibutyltin dilaurilate, copper naphthenate, cobalt naphthenate, zinc naphthenate, triethylamine, 1,4-diazabicyclo[2.2.2]octane and 2,6,7-trimethyl-1,4-diazabicyclo[2.2.2]octane.

These urethanization catalysts may be used singly or may be used in combination of two or more kinds. The adding amount of the urethanization catalyst is in the range of preferably 0.01 to 5 parts by mass, more preferably 0.1 to 1 part by mass, based on 100 parts by mass of the compound (2). If it is less than 0.01 part by mass, reactivity is sometimes lowered. On the other hand, if the adding amount exceeds 5 parts by mass, there is a possibility of occurrence of side reaction during the reaction.

The reaction temperature in the reaction of the compound (1) with the compound (2) is in the range of preferably -10 to 100°C, more preferably 0 to 80°C, still more preferably 10 to 40°C.

By carrying out the reaction under the conditions of the above-mentioned quantity ratio and temperature, side reaction is inhibited, and the urethane compound (A) with good purity can be obtained in favorable yields. Further, since the reaction can be carried out at temperatures in the vicinity of room temperature, the possibility of polymerization of the urethane compounds (A) with each other can be reduced.

### [Curable composition]

The curable composition of the present invention contains the urethane compound (A) obtained by, for example, the above reaction and a polymerization initiator (B).

Since the curable composition of the present invention contains the urethane compound (A), a cured product having an excellent balance between surface hardness, transparency and curling property can be obtained from the curable composition. That is to say, high qualities of various articles comprising the cured products of the present invention can be attained.

The content of the urethane compound (A) in the curable composition of the present invention is in the range of preferably 10 to 99% by mass, more preferably 20 to 99% by mass, still more preferably 30 to 99% by mass. When the content of the urethane compound (A) is in the above range, a curable composition which is excellent in curability and curling property and provides a cured product excellent in pencil hardness, scratch resistance and balance between strength and flexibility can be obtained.

### <(B) Polymerization initiator>

In the present invention, a photopolymerization initiator or a thermal polymerization initiator is employable as the polymerization initiator (B). From the viewpoint of being employable even for a base material with low heat resistance, the photopolymerization initiator is preferable.

When the photopolymerization initiator is used, polymerization reaction of a component contained in the curable composition is brought about by irradiation with active energy rays such as ultraviolet rays or visible rays, whereby a cured product can be obtained.

Examples of such photopolymerization initiators include 1-hydroxycyclohexyl phenyl ketone, 2,2'-dimethoxy-2-phenylacetophenone, xanthone, fluorene, fluorenone, benzaldehyde, anthraquinone, triphenylamine, carbazole, 3-methylacetophenone, 4-chlorobenzophenone, 4,4'-dimethoxybenzophenone, 4,4'-diaminobenzophenone, Michler's ketone, benzoyl propyl ether, benzoin ethyl ether, benzyl dimethyl ketal, 1-(4-isopropylphenyl)-2-hydroxy-2-methylpropan-1-one, 2-hydroxy-2-methyl-1-phenylpropan-1-one, phenylglyoxylic acid methyl ester, thioxanthone, diethylthioxanthone, 2-isopropylthioxanthone, 2-chlorothioxanthone, 2-methyl-1-[4-(methylthio)phenyl]-2-morpholinopropan-1-one, 2,4,6-trimethylbenzoyldiphenylphosphine oxide, 2-benzyl-2-dimethylamino-1-(4-morpholinophenyl)butan-1-one and 1-[4-(2-hydroxyethoxy)-phenyl]-2-hydroxy-2-methylpropan-1-one. These photopolymerization initiators may be used singly or may be used in combination of two or more kinds.

When the thermal polymerization initiator is used, polymerization reaction of a urethane oligomer (C), a reactive monomer (D) and a specific urethane compound (F) described later is brought about by heating, whereby a cured product is obtained.

The thermal polymerization initiator is, for example, an azo compound or an organic peroxide. Examples of the azo compounds include 2,2'-azobis(isobutyronitrile), 2,2'-azobis(2,4-dimethylvaleronitrile), 2,2'-azobis(isobutyric acid)dimethyl, 4,4'-azobis(4-cyanovaleric acid), 2,2'-azobis(2-amidinopropane) dihydrochloride and 2,2'-azobis{2-methyl-N-[2-(1-hydroxybutyl)]-propionamide}. Examples of the organic peroxides include benzoyl peroxide and lauroyl peroxide. These thermal polymerization initiators may be used singly or may be used in combination of two or more kinds.

Although the amount of the polymerization initiator (B) used is not specifically restricted, it is in the range of usually 0.1 to 10 parts by mass, preferably 0.1 to 5 parts by mass, more preferably 0.2 to 2 parts by mass, still more preferably 0.2 to 1 part by mass, based on 10 parts by mass of the urethane compound (A). When the amount of the polymerization initiator (B) used is set within the above range, the rate of polymerization of the urethane compound (A), a urethane oligomer (C), a reactive monomer (D) and a specific urethane compound (F) is increased, and besides, the curable composition is not subject to polymerization inhibition by oxygen or the like.
Moreover, high strength, adhesive strength and heat resistance of the resulting cured film can be attained, and coloring very hardly takes place.

The curable composition of the present invention may further contain a urethane oligomer (C), a reactive monomer (D), a thiol compound (E) and a specific urethane compound (F) in addition to the aforementioned urethane compound (A) represented by the general formula (1) and the polymerization initiator (B). When these are contained, viscosity of the composition can be controlled, and besides, properties of the resulting cured product, e.g., reactivity, mechanical properties such as hardness, elasticity and adhesion, and optical properties such as transparency can be controlled.

### <(C) Urethane oligomer>

The urethane oligomer (C) is an oligomer having a urethane bond, and examples thereof include Beam Set 102, 502H, 505A-6, 510, 550B, 551B, 575, 575CB, EM-90, EM 92 (trade names, available from Arakawa Chemical Industries, Ltd.); Photomer 6008, 6210 (trade names, available from San Nopco Limited); NK Oligo U-2PPA, U-4HA, U-6HA, U-15HA, UA-32P, U-324A, U-4H, U-6H, UA-160TM, UA-122P, UA-2235PE, UA-340P, UA-5201, UA-512 (trade names, Shin-Nakamura Chemical Co., Ltd.); Alonix M-1100, M-1200, M-1210, M-1310, M-1600, M-1960, M-5700, Alon Oxetan OXT-101 (trade names, available from Toagosei Co., Ltd.); AH-600, AT606, UA-306H, UF-8001 (trade names, available from Kyoeisha Chemical Co., Ltd.); Kayarad UX-2201, UX-2301, UX-3204, UX-3301, UX-4101, UX-6101, UX-7101 (trade names, available from Nippon Kayaku Co., Ltd.); Shiko UV-1700B, UV-3000B, UV-6100B, UV-6300B, UV-7000, UV-7600B, UV-7605B, UV-2010B, UV-6630B, UV-7510B, UV-7461TE, UV-3310B, UV-6640B (trade names, available from Nippon Synthetic Chemical Industry Co., Ltd.); Art Resin UN-1255, UN-5200, UN-7700, UN-333, UN-905, HDP-4T, HMP-2, UN-901T, UN-3320HA, UN-3320HB, UN-3320HC, UN-3320HS, H-61, HDP-M20, UN-5500, UN5507 (trade names, available from Negami Chemical Industrial Co., Ltd.); and Ebecryl 6700, 204, 205, 220, 254, 1259, 1290K, 1748, 2002, 2220, 4833, 4842, 4866, 5129, 6602, 8301 (trade names, available from Daicel-UCB Co., Ltd.).

For the purpose of giving hardness to the cured film, the urethane oligomer (C) is preferably a urethane oligomer having 3 or more (meth)acrylate groups, more preferably a urethane oligomer having 6 or more (meth)acrylate groups. Examples of them include the aforesaid U-6HA, U-15HA, UA-32P, UV-1700B, UV-7600B and UV-7605B (trade names). In this specification, the expression "(meth)acrylate" means methacrylate and/or acrylate.

For the purpose of giving flexibility to the cured film, the aforesaid UA-160TM, UA-122P, UA-5201, UV-6630B, UV-7000B, UV-6640B, UN-905, UN-901 and UN-7700 (trade names), etc. can be specifically mentioned as preferred urethane oligomers (C).

Although the weight-average molecular weight of the urethane oligomer (C) is not specifically restricted, it is in the range of preferably 500 to 15000, more preferably 1000 to 3000. In this specification, the weight-average molecular weight means a weight-average molecular weight in terms of polystyrene as measured by GPC.

The urethane oligomers (C) may be used singly or as a mixture of two or more kinds.

Although the amount of the urethane oligomer (C) used is not specifically restricted, it is in the range of usually 0.1 to 100 parts by mass, preferably 0.5 to 80 parts by mass, more preferably 1 to 50 parts by mass, still more preferably 1 to 30 parts by mass, based on 10 parts by mass of the urethane compound (A). By the use of the urethane oligomer (C) in the range, surface hardness of a cured product obtained by curing the curable composition can be controlled, and reduction of curling property during the curing can be made.

### <(D) Reactive monomer>

The reactive monomer (D) is a monomer having a polymerizable unsaturated bond, and may be a monofuncational monomer or a polyfunctional monomer. Specifically, mention may be made of polymerizable unsaturated aromatic compounds, carboxyl group-containing compounds, monofunctional (meth)acrylates, di(meth)acrylates, polyfunctional (meth)acrylates, epoxy poly(meth)acrylates, urethane poly(meth)acrylates and polyester poly (meth) acrylates and the like. These are specifically enumerated below.

Examples of the polymerizable unsaturated aromatic compounds include diisopropenylbenzene, styrene, α-methylstyrene, o-methylstyrene, m-methylstyrene, p-methylstyrene, p-tert-butylstyrene, o-chlorosytene, m-chlorostyrene, p-chlorostyrene, 1,1-diphenylethylene, p-methoxystyrene, N,N-dimethyl-p-aminostyrene, N,N-diethyl-p-aminostyrene, ethylenic unsaturated pyridine and ethylenic unsaturated imidazole.

Examples of the carboxyl group-containing compounds include (meth)acrylic acid, crotonic acid, maleic acid, fumaric acid and itaconic acid.

Examples of the monofunctional (meth)acrylates include alkyl (meth) acrylates such as methyl (meth) acrylate, ethyl (meth) acrylate, propyl (meth)acrylate, isopropyl (meth)acrylate, butyl (meth)acrylate, isobutyl (meth) acrylate, tert-butyl (meth) acrylate, pentyl (meth)acrylate, amyl (meth)acrylate, isoamyl (meth)acrylate, hexyl (meth)acrylate, heptyl (meth)acrylate, octyl (meth)acrylate, isooctyl (meth)acrylate, 2-ethylhexyl (meth)acrylate, nonyl (meth)acrylate, decyl (meth)acrylate, isodecyl (meth)acrylate, undecyl (meth)acrylate, dodecyl (meth)acrylate, lauryl (meth)acrylate, stearyl (meth)acrylate and isostearyl (meth) acrylate;
fluoroalkyl (meth)acrylates such as trifluoroethyl (meth)acrylate, tetrafluoropropyl (meth)acrylate, hexafluoroisopropyl (meth)acrylate, octafluoropentyl (meth)acrylate and heptadecafluorodecyl (meth)acrylate;
hydroxyalkyl (meth)acrylates such as hydroxyethyl (meth)acrylate, hydroxypropyl (meth)acrylate and hydroxybutyl (meth)acrylate;
phenoxyalkyl (meth)acrylates such as phenoxyethyl (meth)acrylate and 2-hydroxy-3-phenoxypropyl (meth)acrylate;
alkoxyalkyl (meth)acrylates such as methoxyethyl (meth)acrylate, ethoxyethyl (meth)acrylate, propoxyethyl (meth)acrylate, butoxyethyl (meth)acrylate and methoxybutyl (meth)acrylate;
polyethylene glycol (meth)acrylates such as polyethylene glycol mono (meth)acrylate, ethoxydiethylene glycol (meth)acrylate, methoxypolyethylene glycol (meth)acrylate, phenoxypolyethylene glycol (meth)acrylate and nonylphenoxypolyethylene glycol (meth)acrylate;
polypropylene glycol (meth)acrylates such as polypropylene glycol mono(meth)acrylate, methoxypolypropylene glycol (meth)acrylate, ethoxypolypropylene glycol (meth)acrylate and nonylphenoxypolypropylene glycol (meth)acrylate;
cycloalkyl (meth) acrylates such as cyclohexyl (meth) acrylate, 4-butylcyclohexyl (meth)acrylate, dicyclopentanyl (meth) acrylate, dicyclopentenyl (meth) acrylate, dicyclopentadienyl (meth) acrylate, bornyl (meth)acrylate, isobornyl (meth) acrylate and tricyclodecanyl (meth) acrylate;
benzyl (meth)acrylate and tetrahydrofurfuryl (meth)acrylate.

Examples of the di(meth)acrylates include ethylene glycol di(meth)acrylate, diethylene glycol di(meth)acrylate, triethylene glycol di(meth)acrylate, polyethylene glycol di(meth)acrylate, propylene glycol di(meth)acrylate, dipropylene glycol di(meth)acrylate, tripropylene glycol di(meth)acrylate, polypropylene glycol di(meth)acrylate, neopentyl glycol di(meth)acrylate, 1,3-propanediol di (meth) acrylate, 1,4-butanediol di(meth)acrylate, 1,6-hexanediol di(meth)acrylate, 1,9-nonanediol di(meth)acrylate, hydroxypivalic acid ester neopentyl glycol di(meth)acrylate, bisphenol A di(meth)acrylate, 2,2-bis(4-(meth)acryloyloxyethoxyphenyl)propane, 2,2-bis(4-(meth)acryloyloxydiethoxyphenyl)propane, trimethylolpropane di(meth)acrylate, tricyclodecane dimethanol diacrylate and bis(2-(meth)acryloyloxyethyl)hydroxyethyl-isocyanurate.

Examples of the polyfunctional (meth)acrylates include trimethylolpropane tri(meth)acrylate, pentaerythritol tri(meth)acrylate, pentaerythritol tetra(meth)acrylate, dipentaerythritol tetra(meth)acrylate, dipentaerythritol penta(meth)acrylate, dipentaerythritol hexa(meth)acrylate, trimethylolpropane trioxyethyl (meth)acrylate and tris(2-hydroxyethyl)isocyanurate tri(meth)acrylate.

Examples of the epoxy poly(meth)acrylates include those obtained by allowing compounds having two or more epoxy groups in a molecule such as bisphenol A type epoxy resin, to react with (meth)acrylic acid or (meth)acrylate having a hydroxyl group.

Examples of the urethane poly (meth) acrylates include urethane di(meth)acrylate obtained by allowing diisocyanate such as 1,6-hexamethylene diisocyanate, isophorone diisocyanate or dicyclohexylmethane diisocyanate, to react with (meth)acrylate having a hydroxyl group such as 2-hydroxyethyl (meth)acrylate; urethane hexa(meth)acrylate obtained by allowing 1, 6-hexamethylene diisocyanate to react with pentaerythritol tri(meth)acrylate; and polyurethane di (meth) acrylate obtained by allowing a urethanization reaction product of dicyclomethane diisocyanate and poly(repeating unit n = 6 to 15) tetramethylene glycol to react with 2-hydroxyethyl (meth)acrylate.

Examples of the polyester poly(meth)acrylates include polyester (meth)acrylate obtained by the reaction of trimethylolpropane with succinic acid and (meth)acrylic acid, and polyester (meth)acrylate obtained by the reaction of trimethylolpropane with ethylene glycol, succinic acid and (meth)acrylic acid.

These compounds having at least one poymerizable unsaturated bond in a molecule can be used singly or in combination of two or more kinds.

Of these reactive monomers (D), polyfunctional (meth) acrylates are preferable, and specifically, trimethylolpropane tri(meth)acrylate, pentaerythritol tetra(meth)acrylate, dipentaerythritol hexa(meth)acrylate and the like are preferable.

Although the amount of the reactive monomer (D) used is not specifically restricted, it is in the range of usually 0.1 to 100 parts by mass, preferably 0.5 to 80 parts by mass, more preferably 1 to 50 parts by mass, still more preferably 1 to 30 parts by mass, based on 10 parts by mass of the urethane compound (A). By the use of the reactive monomer (D) in the above range, surface hardness of a cured product obtained by curing the curable composition can be enhanced.

### <(E) Thiol compound>

The thiol compound (E) is not specifically restricted as long as the compound has a mercapto group in a molecule, but it is preferably a compound having two or more mercapto groups in a molecule.

By incorporating the thiol compound (E) into the curable composition, curability and curling property of the curable composition can be improved. The reason why the curability can be improved is that by the addition of the thiol compound (E), inhibition of radical polymerization by oxygen can be reduced. And the reason why the curling property can be improved is that by the addition of the thiol compound (E), the ene-thiol addition reaction proceeds to thereby depress degree of polymerization and also depress crosslink density. That the curability is excellent means that the amount of energy rays required to cure the curable composition per unit is small. Accordingly, this leads to improvement in productivity of cured products based on the fact that the time of irradiation with energy rays can be shortened and to reduction of cost due to saving of energy, so that the industrial significance of the present invention is extremely great.

Specific examples of the thiol compounds (E) for use in the present invention include ethylene glycol bis(3-mercaptobutyrate), propylene glycol bis(3-mercaptobutyrate), diethylene glycol bis(3-mercaptobutyrate), butanediol bis(3-mercaptobutyrate), octanediol bis(3-mercaptobutyrate), trimethylolpropane tris(3-mercaptobutyrate), pentaerythritol tetrakis(3-mercaptobutyrate), dipentaerythritol hexakis(3-mercaptobutyrate), ethylene glycol bis(2-mercaptopropionate), propylene glycol bis(2-mercaptopropionate), diethylene glycol bis(2-mercaptopropionate), butanediol bis(2-mercaptopropionate), octanediol bis(2-mercaptopropionate), trimethylolpropane tris(2-mercaptopropionate), pentaerythritol tetrakis(2-mercaptopropionate), dipentaerythritol hexakis(2-mercaptopropionate), ethylene glycol bis(3-mercaptoisobutyrate), propylene glycol bis(3-mercaptoisobutyrate), diethylene glycol bis(3-mercaptoisobutyrate), butanediol bis(3-mercaptoisobutyrate), octanediol bis(3-mercaptoisobutyrate), trimethylolpropane tris(3-mercaptoisobutyrate), pentaerythritol tetrakis(3-mercaptoisobutyrate), dipentaerythritol hexakis(3-mercaptoisobutyrate), ethylene glycol bis(2-mercaptoisobutyrate), propylene glycol bis(2-mercaptoisobutyrate), diethylene glycol bis(2-mercaptoisobutyrate), butanediol bis(2-mercaptoisobutyrate), octanediol bis(2-mercaptoisobutyrate),
trimethylolpropane tris(2-mercaptoisobutyrate), pentaerythritol tetrakis(2-mercaptoisobutyrate), dipentaerythritol hexakis(2-mercaptoisobutyrate), ethylene glycol bis(4-mercaptovalerate), propylene glycol bis(4-mercaptoisovalerate), diethylene glycol bis(4-mercaptovalerate), butanediol bis(4-mercaptovalerate), octanediol bis(4-mercaptovalerate), trimethylolpropane tris(4-mercaptovalerate), pentaerythritol tetrakis(4-mercaptovalerate), dipentaerythritol hexakis(4-mercaptovalerate), ethylene glycol bis(3-mercaptovalerate), propylene glycol bis(3-mercaptovalerate), diethylene glycol bis(3-mercaptovalerate), butanediol bis(3-mercaptovalerate), octanediol bis(3-mercaptovalerate), trimethylolpropane tris(3-mercaptovalerate), pentaerythritol tetrakis(3-mercaptovalerate), dipentaerythritol hexakis(3-mercaptovalerate), hydrogenated bisphenol A bis(3-mercaptobutyrate), bisphenol A dihydroxyethyl ether-3-mercaptobutyrate, 4,4'-(9-fluorenylidene)bis(2-phenoxyethyl(3-mercaptobutyrate)), ethylene glycol bis(3-mercapto-3-phenylpropionate), propylene glycol bis(3-mercapto-3-phenylpropionate), diethylene glycol bis(3-mercapto-3-phenylpropionate), butanediol bis(3-mercapto-3-phenylpropionate), octanediol bis(3-mercapto-3-phenylpropionate), trimethylolpropane tris(3-mercapto-3-phenylpropionate), tris-2-(3-mercapto-3-phenylpropionate)ethyl isocyanurate, pentaerythritol tetrakis(3-mercapto-3-phenylpropionate) and dipentaerythritol hexakis(3-mercapto-3-phenylpropionate).

As a more preferred thiol compound (E) for use in the present invention, a compound having two or more structures represented by the following general formula (2) can be mentioned.

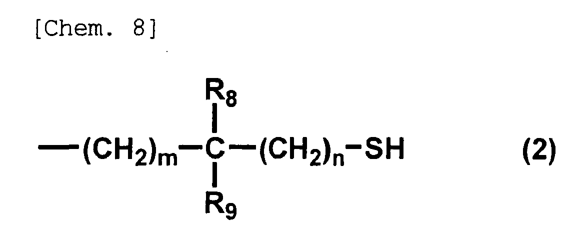

In the general formula (2), R₈ and R₉ are each independently a hydrogen atom or an alkyl or aryl group of 1 to 10 carbon atoms, m is an integer of 0 to 2, n is 0 or 1, and R₈ and R₉ are not hydrogen atoms at the same time.

Examples of the compounds having two or more structures represented by the general formula (2) include polyfunctional thiol compounds represented by the following formulae (6) to (17).

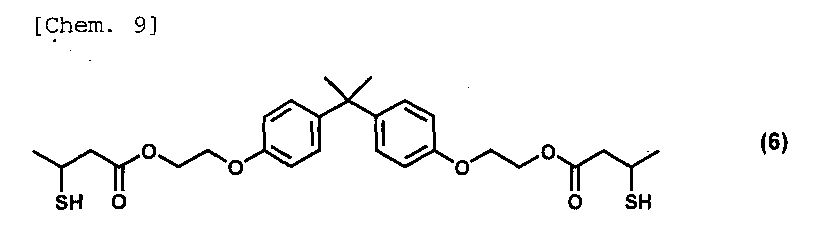

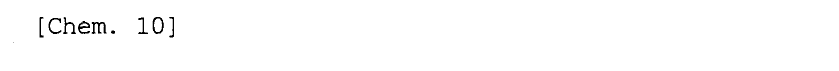

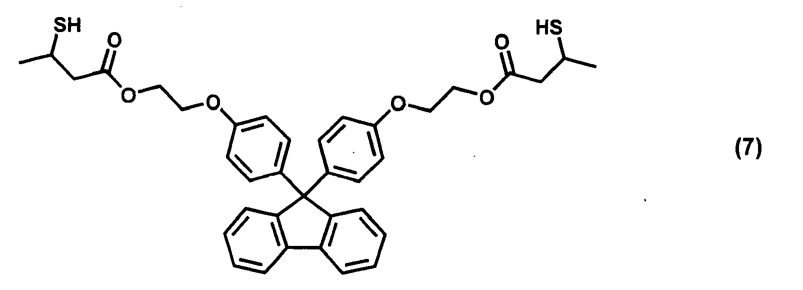

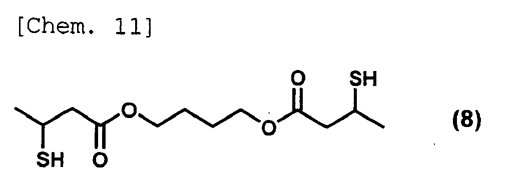

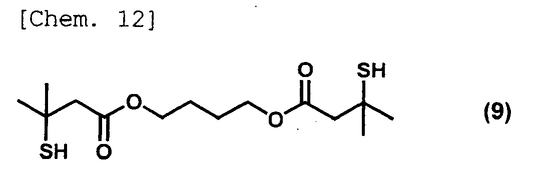

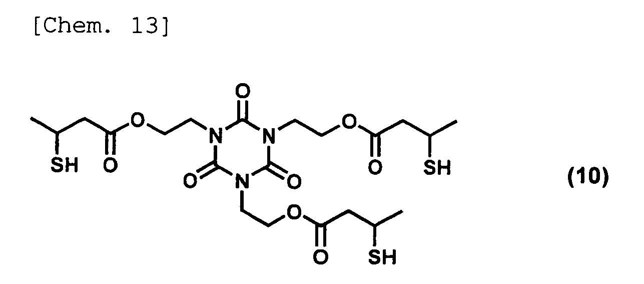

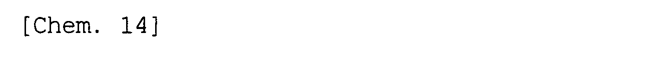

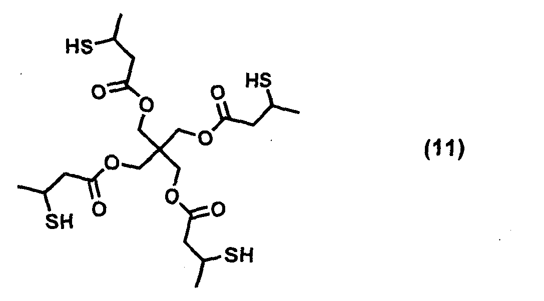

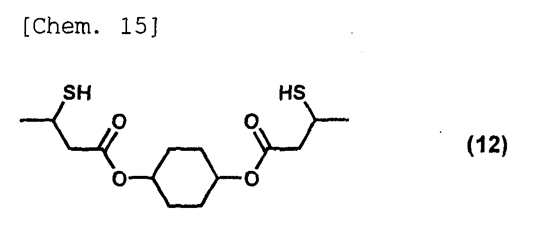

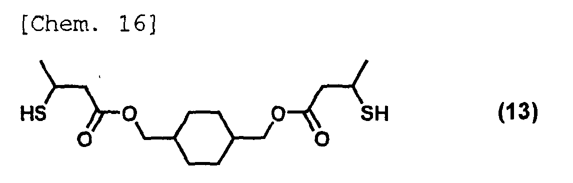

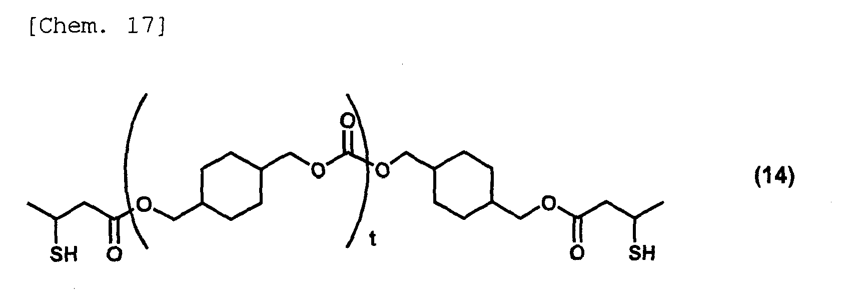

In the formula (14), t is an integer of 1 to 10.

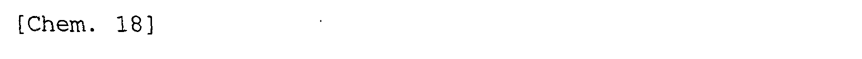

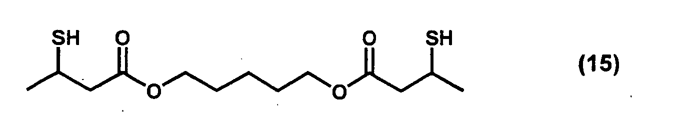

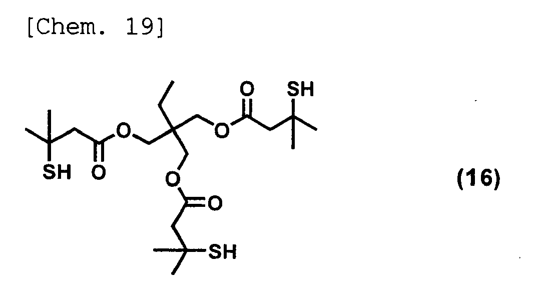

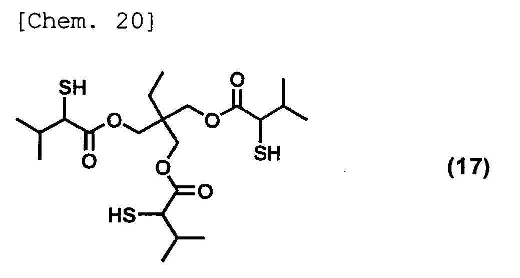

The thiol compounds (E) for use in the present invention can be used singly or in combination of two or more kinds.

Although the molecular weight of the thiol compound (E) for use in the present invention is not specifically restricted, it is in the range of preferably 200 to 2000. If it is less than 200, the curable composition sometimes develops odor, and if it is more than 2000, reactivity and curability of the curable composition are sometimes lowered.

Although the amount of the thiol compound (E) used is not specifically restricted, it is in the range of usually 0.1 to 10 parts by mass, preferably 0.1 to 5 parts by mass, more preferably 0.4 to 1 part by mass, based on 10 parts by mass of the urethane compound (A). When the amount of the thiol compound (E) used is in the above range, curability of the curable composition can be improved. The reason why the curability can be improved is that by the addition of the thiol compound (E), inhibition of radical polymerization by oxygen can be reduced. Moreover, properties of the resulting cured product, e.g., reactivity, mechanical properties such as hardness, elasticity and adhesion, and optical properties such as transparency, can be controlled.

### <(F) Specific urethane compound>

The specific urethane compound (F) (also referred to as a "Urethane compound (F) " simply hereinafter) which may be used in the present invention is represented by the following general formula (3), and has two or more polymerizable unsaturated bonds in a molecule.

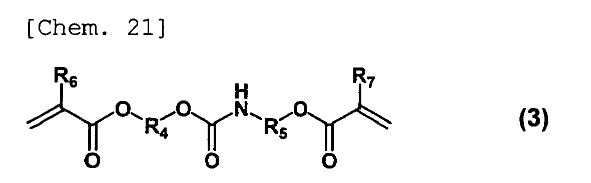

In the general formula (3), R₄ is a straight-chain or branched divalent aliphatic group of 1 to 12 carbon atoms, a divalent organic group of 3 to 12 carbon atoms having an alicyclic group, a divalent organic group having an aromatic ring or [-(CH₂)_{g}-O-(CH₂)ₕ-]ᵢ (g and h are each independently an integer of 1 to 10, and i is an integer of 1 to 5).

From the viewpoint of curability of the curable composition and the viewpoint of hardness of a cured product obtained by curing the curable composition (also referred to as a "cured product" simply hereinafter), R₄ is preferably a divalent aliphatic group of 1 to 6 carbon atoms or [-(CH₂)_{g}-O-(CH₂)ₕ-]ᵢ (g and h are each independently an integer of 1 to 5, and i is an integer of 1 to 3), more preferably a divalent aliphatic group of 2 to 4 carbon atoms or [-(CH₂)_{g}-O-(CH₂)ₕ-]ᵢ (g and h are each independently an integer of 2 to 4, and i is 1 or 2), still more preferably a divalent aliphatic group of 2 carbon atoms or -(CH₂)₂-O-(CH₂)₂-.

In the general formula (3), R₅ is a straight-chain or branched divalent aliphatic group of 1 to 10 carbon atoms, a divalent organic group of 3 to 10 carbon atoms having an alicyclic group, a divalent organic group having an aromatic ring or [-(CH₂)ₚ-O-(CH₂)_{q}-]ᵣ (p and q are each independently an integer of 1 to 10, and r is an integer of 1 to 5).

From the viewpoint of curability of the curable composition and the viewpoint of hardness of a cured product obtained by curing the curable composition, R₅ is preferably a divalent aliphatic group of 1 to 6 carbon atoms or [-(CH₂)ₚ-O-(CH₂)_{q}-]ᵣ (p and q are each independently an integer of 1 to 5, and r is an integer of 1 to 3), more preferably a divalent aliphatic group of 2 to 4 carbon atoms or [-(CH₂)ₚ-O-(CH₂)_{q}-]ᵣ (p and q are each independently an integer of 2 to 4, and r is 1 or 2), still more preferably a divalent aliphatic group of 2 carbon atoms or -(CH₂)₂-O-(CH₂)₂-.

In the general formula (3), R₆ and R₇ are each independently a hydrogen atom or a methyl group. From the viewpoint of being cured in a small exposed dose, they are each preferably a hydrogen atom.

Although the content of the urethane compound (F) in the curable composition of the present invention is not specifically restricted, it is in the range of 0.1 to 100 parts by mass, preferably 0.5 to 80 parts by mass, more preferably 1 to 50 parts by mass, still more preferably 1 to 30 parts by mass, based on 10 parts by mass of the urethane compound (A). When the amount of the urethane compound (F) used is set within the above range, viscosity of the curable composition can be lowered, and coating property can be enhanced. Further, the polymerization rate of the curable composition can be increased, and besides, surface hardness of the cured product can be enhanced.

### <Other components>

In the curable composition of the present invention, a polymerization inhibitor may be contained in an amount of not more than 0.1 part by mass based on 100 parts by mass of the curable composition. The polymerization inhibitor is used in order to prevent a component contained in the curable composition from initiating polymerization reaction during storage. Examples of the polymerization inhibitors include hydroquinone, hydroquinone monomethyl ether, benzoquinone, p-t-butyl catechol and 2,6-di-t-butyl-4-methylphenol.

To the curable composition of the present invention, a leveling agent, a filler, a pigment, an inorganic filler, a solvent, and other modifiers may be added.

Examples of the leveling agents include a polyether modified dimethylpolysiloxane copolymerization product, a polyester modified dimethylpolysiloxane copolymerization product, a polyether modified methylalkyl polysiloxane copolymerization product, an aralkyl modified methylalkyl polysiloxane copolymerization product and a polyether modified methylalkyl polysiloxane copolymerization product.

Examples of the fillers or the pigments include calcium carbonate, talc, mica, clay, silica (colloidal silica, Aerosil (trademark), etc.), barium sulfate, aluminum hydroxide, zinc stearate, zinc white, red iron oxide and azo pigment.

Examples of the inorganic fillers include conductive metal fine particles and conductive metal oxide fine particles. Examples of the metals include gold,silver,copper,platinum,aluminum,antimony, selenium, titanium, tungsten, tin, zinc, indium and zirconium. And examples of the metal oxides include alumina, antimony oxide, selenium oxide, titanium oxide, tungsten oxide, tin oxide, antimony doped tin oxide (ATO (tin oxide doped with antimony)), phosphorus doped tin oxide, zinc oxide, zinc antimonate and tin doped indium oxide.

Examples of the other modifiers include natural and synthetic high-molecular substances for example, polyolefin-based resin, chlorinated modified polyolefin-based resin, unsaturated polyester resin, vinyl ester resin, vinyl urethane resin, vinyl ester urethane resin, polyisocyanate, polyepoxide, epoxy-terminated polyoxazolidone, acrylic resins, alkyd resins, urea resins, melamine resins, polydiene-based elastomer, saturated polyesters, saturated polyethers, cellulose derivatives such as nitrocellulose and cellulose acetate butyrate, and fats and oils such as linseed oil, wood oil, soybean oil, castor oil and epoxidized oil.

The curable composition of the present invention can be formulated and prepared by mixing the urethane compound (A) of the present invention represented by the general formula (1), the polymerization initiator (B), and if necessary, the urethane oligomer (C), the reactive monomer (D), the thiol compound (E) and the urethane compound (F) by a mixing machine such as a mixer, a ball mill or a three-roll mill, at room temperature or under the heating conditions, or by adding a reactive monomer or a solvent as a diluent to dissolve them. An example of the reactive monomer used herein as the diluent is the aforesaid reactive monomer (D).

Specific examples of the solvents for use in the present invention include esters such as ethyl acetate, butyl acetate and isopropyl acetate;
ketones such as acetone, methyl ethyl ketone, methyl isobutyl ketone and cyclohexanone;
cyclic ethers such as tetrahydrofuran and dioxane;
amides such as N,N-dimethylformamide;
aromatic hydrocarbons such as toluene, and halogenated hydrocarbons such as methylene chloride;
ethylene glycols such as ethylene glycol, ethylene glycol methyl ether, ethylene glycol mono-n-propyl ether, ethylene glycol monomethyl ether acetate, diethylene glycol, diethylene glycol monomethyl ether, diethylene glycol monoethyl ether and diethylene glycol monoethyl ether acetate; and
propylene glycols such as propylene glycol, propylene glycol methyl ether, propylene glycol ethyl ether, propylene glycol butyl ether, propylene glycol propyl ether, propylene glycol monomethyl ether acetate, dipropylene glycol, dipropylene glycol monomethyl ether, dipropylene glycol monoethyl ether and dipropylene glycol monomethyl ether acetate. Of these, preferable are ethyl acetate, methyl ethyl ketone, cyclohexanone, toluene, dichloromethane, diethylene glycol monomethyl ether and propylene glycol monomethyl ether acetate.

The above solvents can be used singly or in combination of two or more kinds.

Although the amount of the solvent used is not specifically restricted, it is in the range of usually 5 to 20 parts by mass, preferably 5 to 10 parts by mass, based on 10 parts by mass of the curable composition.

The curable composition of the present invention can be cured by, for example, applying the curable composition onto a base material to form a coating film and then irradiating it with active energy rays or heating it. For the curing, both of irradiation with active energy rays and heating may be carried out.

Examples of the coating methods include coating with a bar coater, an applicator, a die coater, a spin coater, a spray coater, a curtain coater, a roll coater or the like, coating by screen printing or the like and coating by dipping or the like.

The amount of the curable composition of the present invention coated onto the base material is not specifically restricted, and can be appropriately adjusted according to a purpose. It is preferably such an amount that the film thickness of the coating film for the purpose of evaluation which is obtained after curing treatment by irradiation with active energy rays after coating and drying becomes 1 to 200 µm, more preferably 5 to 100 µm.

As the active energy rays used for curing, electron rays or lights in the ultraviolet to infrared wavelength region are preferable. As the light sources, for example, when the active energy rays are ultraviolet rays, an extra-high pressure mercury light source or a metal halide light source is employable, when the active energy rays are visible rays, a metal halide light source or a halogen light source is employable, and when the active energy rays are infrared rays, a halogen light source is employable. In addition, other light sources such as laser and LED are employable. The irradiation dose of the active energy rays is properly set according to the type of the light source, film thickness of the coating film, etc.

### EXAMPLES

The present invention is further described with reference to the following examples and comparative examples in detail, but the present invention is in no way limited to the description of them.

The term "part(s)" in the examples means "part(s) by mass".

### (Synthesis Example 1)

### [Urethane compound (A-1)]

Into a reaction vessel, 300 parts of dichloromethane (available from Junsei Chemical Co., Ltd.), 20 parts of tris(2-hydroxyethyl)isocyanurate (available from Nissan Chemical Industries, Ltd.) as a compound having a hydroxyl group and an isocyanurate ring structure in a molecule and 0.7 parts of dibutyltin dilaurate (available from Tokyo Kasei Kogyo Co., Ltd.) were charged and stirred. Thereafter, 33 parts of 2-acryloyloxyethyl isocyanate (available from Showa Denko K. K. , trade name: Karenz AOI (registered trademark)) were dropwise added slowly, followed by stirring at room temperature. After it was confirmed by high performance liquid chromatography that the peak of tris (2-hydroxyethyl)isocynurate had nearly disappeared, the reaction was completed. Subsequently, by the use of 203 parts of hexane containing 200 ppm of 2,6-di-tert-butyl-4-methylphenol (BHT, available from Junsei Chemical Co. , Ltd.), washing was carried out 4 times to obtain a urethane compound (A-1).

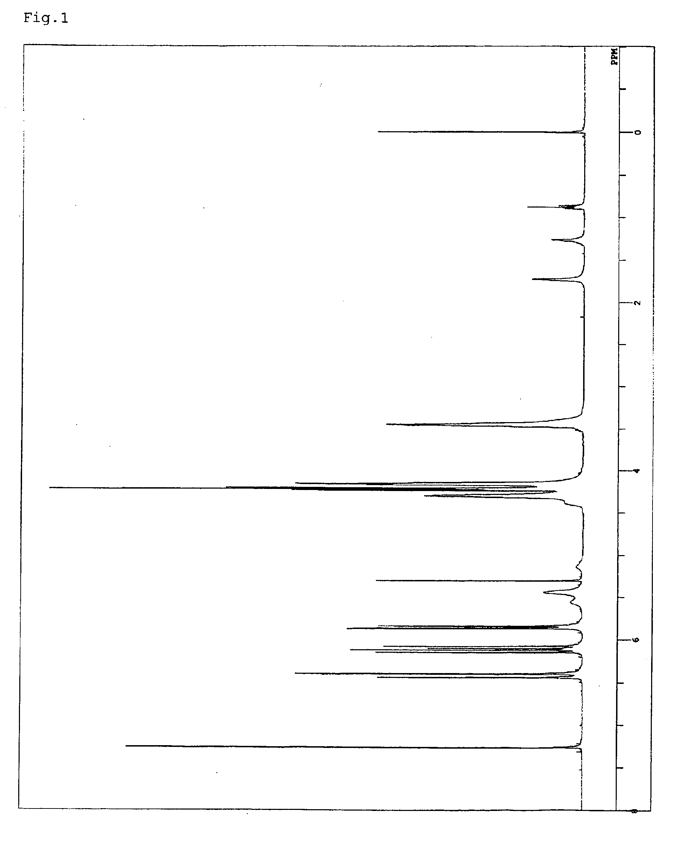

A ¹H-NMR chart of the urethane compound (A-1) is shown in Fig. 1. The ¹H-NMR was carried out in deuterated chloroform by the use of AMX400 available from Bruker. The assignment of the ¹H-NMR chart is shown below.

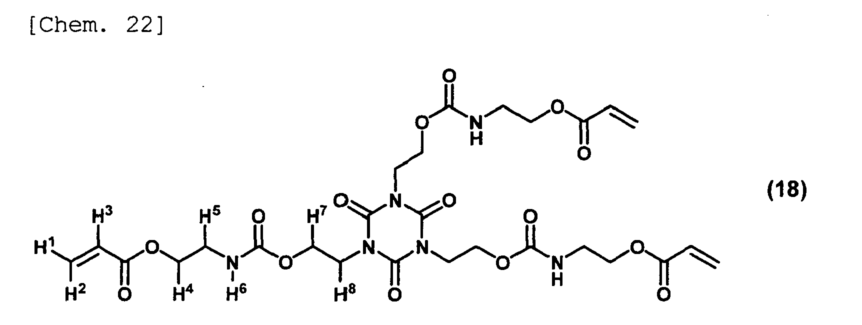

3.45 ppm: H⁵
4.15-4.30 ppm: H⁴, H⁷, H⁸
5.44 ppm: H⁶
5.86 ppm: H¹
6.12 ppm: H³
6.42 ppm: H²

### (Synthesis Example 2)

### [Urethane compound (A-2)]

A urethane compound (A-2) was obtained in the same manner as in Synthesis Example 1, except that 2-methacryloyloxyethyl isocyanate (available from Showa Denko K.K., trade name: Karenz MOI (registered trademark)) was used instead of 2-acryloyloxyethyl isocyanate.

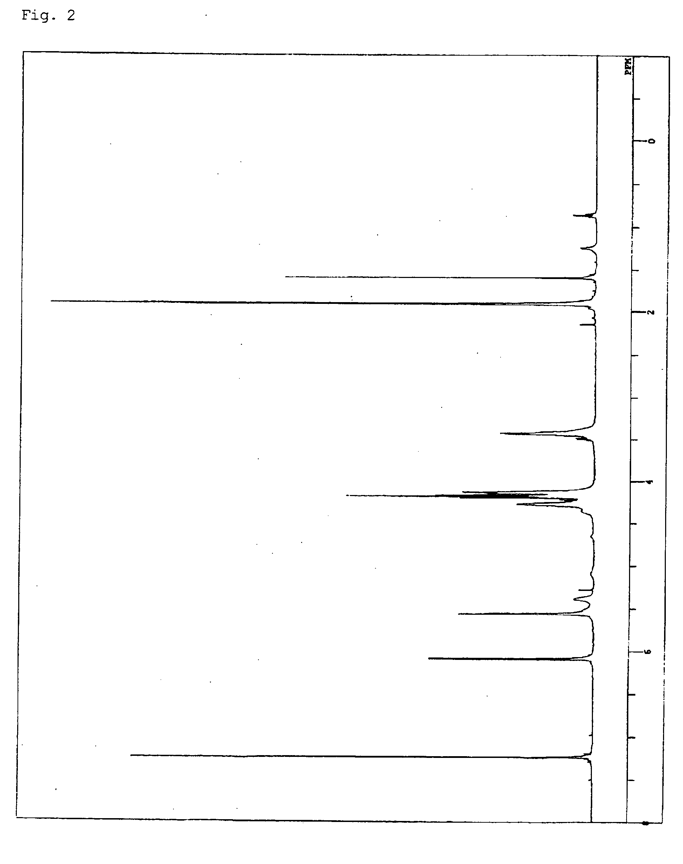

A ¹H-NMR chart of the urethane compound (A-2) is shown in Fig. 2. The ¹H-NMR was carried out in deuterated chloroform by the use of AMX400 available from Bruker. The assignment of the ¹H-NMR chart is shown below.

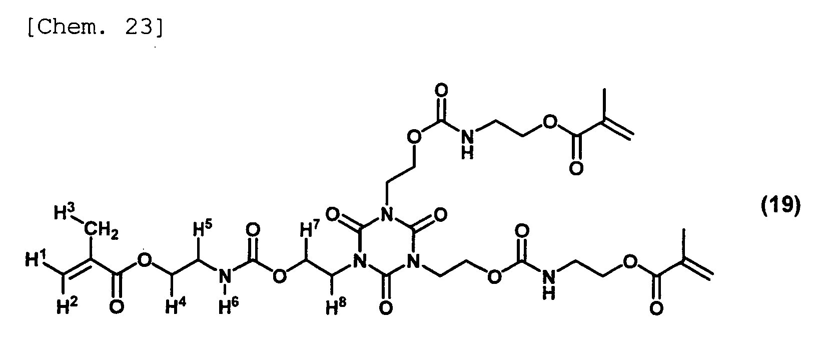

1.92 ppm: H³
3.43-3.47 ppm: H⁵
4.13-4.28 ppm: H⁴, H⁷, H⁸
5.39 ppm: H⁶
5.56 ppm: H¹
6.09 ppm: H²

### (Synthesis Example 3)

### [Urethane compound (A-3)]

A urethane compound (A-3) was obtained in the same manner as in Synthesis Example 1, except that 46 parts of 2-(2-methacryloyloxyethyloxy)ethylisocyanate (available from Showa Denko K.K., trade name: Karenz MOI (registered trademark) EG) were used instead of 2-acryloyloxyethyl isocyanate.

### (Synthesis Example 4)

### [Urethane compound (F-1)]

Into a reaction vessel, 100 parts of 2-hydroxyethyl acrylate (available from Osaka Organic Chemical Industry Ltd.), 142 parts of hexane (available from Junsei Chemical Co., Ltd.) containing 200 ppm of 2,6-di-tert-butyl-4-methylphenol (BHT, available from Junsei Chemical Co. , Ltd.) and 2.8 parts of dibutyltin dilaurate (available from Tokyo Kasei Kogyo Co., Ltd.) were charged and stirred. Thereafter, 122 parts of 2-acryloyloxyethyl isocyanate (available from Showa Denko K.K., trade name: Karenz AOI (registered trademark)) were dropwise added slowly, followed by stirring at room temperature. After it was confirmed by high performance liquid chromatography that the peak of 2-hydroxyethyl acrylate had nearly disappeared, the reaction was completed. Subsequently, by the use of 203 parts of hexane containing 200 ppm of BHT, washing was carried out 4 times to obtain a urethane compound (F-1).

### (Synthesis Example 5)

### [Urethane compound (G-1)]

Into a reaction vessel, 300 parts of toluene containing 200 ppm of 2, 6-di-tert-butyl-4-methylphenol (BHT, available from Junsei Chemical Co., Ltd.), 100 parts of BPX-33 (available from ADEKA CORPORATION) as bisphenol type polyol, 76 parts of isophorone diisocyanate (available from Tokyo Kasei Kogyo Co., Ltd.) as polyisocyanate, 40 parts of 2-hydroxyethyl acrylate (available from Osaka Organic Chemical Industry Ltd.) and 0.054 parts of dibutyltin dilaurate (available from Tokyo Kasei Kogyo Co., Ltd.) were charged and allowed to react at 70°C for 10 hours. Thereafter, by the use of 200 parts of hexane containing 200 ppm of BHT, washing was carried out 4 times to obtain a urethane compound (G-1).

### (Synthesis Example 6)

### [Urethane compound (G-2)]

Into a reaction vessel, 100 parts of isophorone diisocyanate (available fromTokyo Kasei Kogyo Co. , Ltd.), 0.141 parts of dibutyltin dilaurate (available from Tokyo Kasei Kogyo Co., Ltd.) and 0.073 parts of 2,6-di-tert-butyl-4-methylphenol (BHT, available from Junsei Chemical Co., Ltd.) were charged and stirred at 60°C for 1 hour. Thereafter, 105 parts of 2-hydroxyethyl acrylate (available fromOsaka Organic Chemical Industry Ltd.) were added, followed by stirring at 80°C for 2 hours. By the use of 200 parts of hexane containing 200 ppm of BHT, washing was carried out 4 times to obtain a urethane compound (G-2).

### (Example 1)

### [Preparation of curable composition]

In a container shielded from ultraviolet rays, 98 parts of the urethane compound (A-1) prepared in Synthesis Example 1, 2 parts of 2-methyl-1-[4-(methylthio)phenyl]-2-morpholinopropan-1-one (available from Ciba Specialty Chemicals Inc., trade name: Irgacure 907) as a photopolymerization initiator and 100 parts of dichloromethane as a solvent were blended, and they were stirred and mixed at room temperature to homogeneously dissolve, whereby a curable composition solution (solution 1) was obtained.

### (Example 2)

### [Preparation of curable composition]

A curable composition solution (solution 2) was obtained in the same manner as in Example 1, except that (A-2) was used instead of (A-1).

### (Comparative Example 1)

### [Preparation of curable composition]

A curable composition solution (solution 3) was obtained in the same manner as in Example 1, except that tris[2-(acryloyloxy)ethyl]isocyanurate (THI-AC, available from Aldrich Co.) was used instead of (A-1).

### (Comparative Example 2)

### [Preparation of curable composition]

A curable composition solution (solution 4) was obtained in the same manner as in Example 1, except that trimethylolpropane triacrylate (TMP-3A, available from Kyoeisha Chemical Co., Ltd.) was used instead of (A-1).

### (Comparative Example 3)

### [Preparation of curable composition]

A curable composition solution (solution 5) was obtained in the same manner as in Example 1, except that trimethylolpropane trimethacrylate (TMP-3MA, available from Kyoeisha Chemical Co., Ltd.) was used instead of (A-1).

### [Preparation of cured film]

Each of the curable composition solutions (solution 1) to (solution 5) prepared in the above Examples 1 and 2 and Comparative Examples 1 to 3 was coated onto different glass substrates (50mm × 50mm) such that the thickness of the cured film would become 100 µm. Subsequently, the coating films were exposed to light at 3 J/cm² by the use of an exposure device into which an extra-high pressure mercury lamp was incorporated, so that the coating films were cured.

### [Property evaluation methods]

### (1) Pencil hardness

The cured films obtained in the above [Preparation of cured film] were scratched with Uni (registered trademark) available from Mitsubishi Pencil Co., Ltd. in such a manner that the angle between the pencil and the cured film was 45 degrees, then a pencil of maximum hardness which made no scratch mark was determined, in accordance with JIS-K5600. The hardness was regarded as a pencil hardness and is set forth in Table 1.

### (2) Light transmittance

The cured films obtained in the [Preparation of cured film] weremeasured regarding the transmittance for light having a wavelength of 400 nm by the use of a spectrophotometer (available from JASCO Corporation, UV3100) in accordance with JIS-K7105. The results are set forth in Table 1.

### (3) Curling property

Each of the curable composition solutions (solution 1) to (solution 5) was coated onto different polyimide films of 3 cm square size such that the thickness of the cured film would become 40 µm. Subsequently, they were exposed to light at 500 mJ/cm² by an exposure device into which an extra-high pressure mercury lamp was incorporated, then the heights of respective four sides which warped up on a horizontal pedestal were measured. Their mean value was regarded as a value of curling property. The results are set forth in Table 1. The nearer to 0 mm it is, the better the curling property is.

### (4) Light-exposed dose for polymerization initiation (Curability)

Curable composition solutions (test solutions 1 to 5) were obtained in the same manners as in each of Examples 1 and 2 and Comparative Examples 1 to 3, except that 1 part of 1-hydroxycyclohexyl phenyl ketone (available from Ciba Specialty Chemicals Inc., trade name: Irgacure 184) was used instead of 2 parts of 2-methyl-1-[4-(methylthio)phenyl]-2-morpholinopropan-1-one.

Under irradiation with UV of 16 mW, viscosity changes of the curable composition solutions (test solutions 1 to 5) were measured by the use of a rheometer with an UV cure measuring cell (rheometer (VAR·DAR) available from Reologica Co.). On the assumption that the polymerization reaction was initiated at the time the viscosity of the curable composition solution became 200 Pa·s, the light-exposed dose for polymerization initiation was determined. The results are set forth in Table 2. As the light-exposed dose for polymerization initiation is decreased, the curability becomes better.

**Table 1**

| | | Ex. 1 | Ex. 2 | Comp. Ex. 1 | Comp. Ex. 2 | Comp. Ex. 3 |
|---|---|---|---|---|---|---|
| Composition | A-1 | 98 | | | | |
| | A-2 | | 98 | | | |
| | THI-AC | | | 98 | | |
| | TMP-3A | | | | 98 | |
| | TMP-3MA | | | | | 98 |
| | Irgacure 907 | 2 | 2 | 2 | 2 | 2 |
| Evaluation | Pencil hardness | 6H | 6H | 3H | 6H | 7H |
| | Transmittance (%) | 97.3 | 98.9 | 96.7 | 96.4 | 95.5 |
| | Curling property (mm) | +5.4 | +5.0 | +6.8 | +10.5 | +9.8 |

| | | | | | | |
|---|---|---|---|---|---|---|
| *The unit of each numerical value in the formulation of the composition is part (s) by mass, and the composition contains 100 parts of a solvent (dichloromethane). | | | | | | |

**Table 2**

| | Test solution 1 | Test solution 2 | Test solution 3 | Test solution 4 | Test solution 5 |
|---|---|---|---|---|---|
| Light-exposed dose for polymerization initiation (mJ/cm²) | 15.4 | 99.8 | 12.5 | 16.3 | 103.7 |

| | | | | | |
|---|---|---|---|---|---|
| * The unit of each numerical value in the formulation of the composition is part(s) by mass. | | | | | |

It canbe seen fromTable 1 and Table 2 that the curable composition having the component (A-1) of the present invention was excellent in curability, and the cured film prepared by the use thereof was excellent in pencil hardness, transparency and curling property. As compared with this, the cured film of Comparative Example 1 containing the THI-AC component having an acrylate structure similarly to the component (A-1) was inferior in pencil hardness. The cured film of Comparative Example 2 containing the TMP-3A component likewise having an acrylate structure was inferior in curling property.

The cured film prepared by the use of the curable composition having the component (A-2) was excellent in pencil hardness, transparency and curling property. In contrast with this, the curable composition of Comparative Example 3 containing the TMP-3MA component having a methacrylate structure similarly to the component (A-2) was inferior in curability. And it was inferior in transparency and curling property though it was excellent in pencil hardness, resulting in a bad balance of physical properties.

### (Examples 3 to 9)

### [Preparation of curable composition]

In a container shielded from ultraviolet rays, the components (A) to (F) shown in Table 3 were stirred and mixed at room temperature in accordance with the formulation compositions (unit: part(s) by mass) shown in Table 3 to homogeneously dissolve, whereby curable composition solutions were prepared.

### (Comparative Examples 4 to 7)

### [Preparation of curable composition]

In a container shielded from ultraviolet rays, the components (B), (D) and (G) shown in Table 4 were stirred and mixed at room temperature in accordance with the formulation compositions (unit: part (s) by mass) shown in Table 4 to homogeneously dissolve, whereby curable composition solutions were prepared.

### [Preparation of cured film]

Each of the curable composition solutions of Examples 3 to 9 and Comparative Examples 4 to 7 shown in Table 3 and Table 4 was coated onto different glass substrates (50mm×50mm) such that the thickness of the cured film would become 100 µm. Subsequently, the coating films were exposed to light at 1 J/cm² by an exposure device into which an extra-high pressure mercury lamp was incorporated, so that the coating films were cured.

### [Property evaluation methods]

### (1) Pencil hardness

The cured films obtained in the [Preparation of cured film] were scratched with Uni (registered trademark) available from Mitsubishi Pencil Co., Ltd. in such a manner that the angle between the pencil and the cured film was 45 degrees, then a pencil of maximum hardness which made no scratch mark was determined, in accordance with JIS-K5600. The hardness was regarded as a pencil hardness and is set forth in Table 3 and Table 4.

### (2) Light transmittance

The cured films obtained in the [Preparation of cured film] were measured regarding the transmittance for light having a wavelength of 400 nm by the use of a spectrophotometer (available from JASCO Corporation, UV3100) in accordance with JIS-K7105. The results are set forth in Table 3 and Table 4.

### (3) Curling property

Each of the curable composition solutions obtained in the [Preparation of cured film] was coated onto different polyimide films of 3 cm square size such that the thickness of the cured film would become 40 µm. Subsequently, they were exposed to light at 500 mJ/cm² by an exposure device into which an extra-high pressure mercury lamp was incorporated. The heights of respective four sides having warped up on a horizontal pedestal were measured, and their mean value was regarded as a value of curling property. The results are set forth in Table 3 and Table 4. The nearer to 0 mm it is, the better the curling property is.

### (4) Light- exposed dose for polymerization initiation (curability)

Under irradiation with UV of 16 mW, viscosity changes of the curable composition solutions obtained in the [Preparation of curable composition] were measured by the use of a rheometer with an UV cure measuring cell (rheometer (VAR·DAR) available from Reologica Co.). On the assumption that the polymerization reaction was initiated at the time the viscosity of the curable composition solution became 200 Pa·s, the light-exposed dose for polymerization initiation was determined. The results are set forth in Table 3 and Table 4. As the light-exposed dose for polymerization initiation is decreased, the curability becomes better.

### (5) Scratch resistance

The surfaces of the cured films obtained in the [Preparation of cured film] were each rubbed back and forth 10 times with a steel wool of #1000 at a stroke of 25mmand a rate of 30mm/sec under application of a load of 175 g/cm². Thereafter, presence of scratch mark on the surface was visually observed.

The evaluation criteria are as follows, and the evaluation results are set forth in Table 3 and Table 4.
A: No scratch mark is observed.
B: A small number of scratch marks are observed.
C: A large number of scratch marks are observed.

### (6) Bending resistance

The cured films obtained in the [Preparation of cured film] were each wound around a cylindrical metal bar having a diameter of 2 mm, and presence of crack was visually observed.

The evaluation criteria are as follows, and the evaluation results are set forth in Table 3 and Table 4.

A: No crack is formed.
B: Cracks are rarely formed.
C: Cracks are inevitably formed.

### (7) Elongation at break (flexibility)

The cured films obtained in the [Preparation of cured film] were each cut into a strip (5mm×30mm), and 7.5 mm of both ends thereof were applied tabs with polyimide films.

They were pulled by the use of a small table tester (EZ-test, available from Shimadzu Corporation) under the conditions of a gap distance of 15 mm and a pulling rate of 5 mm/min in accordance with JIS-K7127 to measure an elongation at break. The evaluation results are set forth in Table 3 and Table 4. The elongation at break indicates flexibility, and when it is high, a higher flexibility is indicated.

**Table 3**

| | | | Ex.3 | Ex.4 | Ex.5 | Ex.6 | Ex.7 | Ex.8 | Ex.9 |
|---|---|---|---|---|---|---|---|---|---|
| Composition | (A) | A-1 | 50 | 26 | | | | | |
| | (A) | A-3 | | | 70 | 75 | 85 | 75 | 75 |
| | (B) | Irgacure 184 | 3 | 3 | 3 | 5 | 5 | 5 | 5 |
| | (C) | UA-122P | 11 | 11 | | | | | |
| | | U-15HA | 25 | 25 | | | | | 15 |
| | (D) | PE-4A | 39 | 39 | | | | | |
| | | DPHA | | | 5 | 15 | 15 | 25 | |
| | (E) | BD1 | 50 | 5 | | | | | |
| | (F) | F-1 | | 24 | 25 | 10 | | | 10 |
| Evaluation | | Pencil hardness | 2H | 2H | 2H | 2H | 2H | 3H | 2H |
| | | Transmittance (%) | 97.5 | 97.1 | 98.3 | 97.4 | 97.0 | 97.4 | 97.4 |
| | | Curling property (mm) | +4.3 | +8.7 | +2.9 | +2.6 | +2.1 | +2.3 | +2.4 |
| | | Light-exposed dose for polymerization initiation (mJ/cm²) | <1.0 | <1.0 | 20.2 | 7.7 | 13.4 | 12.5 | 7.0 |
| | | Scratch resistance | B | A | B | B | B | B | B |
| | | Bending resistance | B | B | A | A | A | B | A |
| | | Elongation at break (%) | 3.4 | 2.1 | 14.8 | 11.4 | 7.6 | 7.6 | 10.5 |

| | | | | | | | | | |
|---|---|---|---|---|---|---|---|---|---|
| *The composition contains 100 parts of a solvent (dichloromethane). | | | | | | | | | |

Details of the abbreviations in Table 3 are given below.

A-1: Urethane compound obtained in Synthesis Example 1
A-3: Urethane compound obtained in Synthesis Example 3
Irgacure 184: available from Ciba Specialty Chemicals Inc., 1-hydroxycyclohexyl phenyl ketone, trade name: Irgacure 184, photopolymerization initiator
UA-122P: available from Shin-Nakamura Chemical Co., Ltd., trade name: UA-122P, urethane oligomer
U-15HA: available from Shin-Nakamura Chemical Co. , Ltd., trade name: U-15HA, urethane oligomer
PE-4A: available from Kyoeisha Chemical Co., Ltd. , trade name: PE-4A, acrylic monomer
DPHA: available from Kyoeisha Chemical Co., Ltd. , trade name: DPHA, acrylic monomer
BD1: available from Showa Denko K.K., 1,4-bis(3-mercaptobutyryloxy)butane, trade name: Karenz (registered trademark) MT BD1), thiol compound
F-1: urethane compound obtained in Synthesis Example 4

**Table 4**

| | | | Comp. Ex. 4 | Comp. Ex. 5 | Comp. Ex. 6 | Comp. Ex. 7 |
|---|---|---|---|---|---|---|
| Composition | (B) | Irgacure 184 | 4 | 5 | 5 | 5 |
| | (D) | AMP-60G | 25 | | | |
| | | AMP-10G | | 20 | 15 | |
| | | PE-3A | | | 5 | |
| | | DPHA | | | | 20 |
| | (G) | G-1 | 75 | | | |
| | | G-2 | | 80 | 80 | 80 |
| Evaluation | | Pencil hardness | 8B | B | HB | H |
| | | Transmittance (%) | 98.5 | 98.5 | 98.4 | 98.3 |
| | | Curling property (mm) | +1.0 | +3.1 | +3.9 | +4.6 |
| | | Light-exposed dose for polymerization initiation (mJ/cm²) | 3.8 | <1.0 | <1.0 | 1.0 |
| | | Scratch resistance | C | C | C | C |
| | | Bending resistance | A | A | A | B |
| | | Elongation at break (%) | 64.5 | 8.8 | 10.3 | 4.6 |

| | | | | | | |
|---|---|---|---|---|---|---|
| *The composition contains 100 parts of a solvent (dichloromethane). | | | | | | |

Details of the abbreviations in Table 4 are given below.

Irgacure 184: available from Ciba Specialty Chemicals Inc., 1-hydroxycyclohexyl phenyl ketone, trade name: Irgacure 184, photopolymerization initiator
AMP-60G: available from Shin-Nakamura Chemical Co. , Ltd. , trade name: AMP-60G, acrylic monomer
AMP-10G:available from Shin-Nakamura Chemical Co., Ltd., trade name: AMP-10G, acrylic monomer
PE-3A: available from Kyoeisha Chemical Co., Ltd., trade name: PE-3A, acrylic monomer
DPHA: available from Kyoeisha Chemical Co., Ltd., trade name: DPHA, acrylic monomer
G-1: Urethane compound obtained in Synthesis Example 5
G-2: Urethane compound obtained in Synthesis Example 6
From Table 3 and Table 4, it can be seen that the curable compositions of the present invention (Examples 3 and 4) having the component (A-1) had very good curability and could form cured films which were excellent in scratch resistance and bending resistance and had strength and flexibility compatible with each other.

On the other hand, the curable resins prepared using the curable compositions of the present invention having the component (A-3) were particularly excellent in curling property and bending resistance and also had good scratch resistance, as shown in Examples 5 to 9.

In contrast with them, the curable composition (Comparative Example 4) having almost the same composition as that of a curable composition used in Example 1 of the patent document 5 was inferior in the pencil hardness and the scratch resistance though it had sufficient curling property, bending resistance and elongation at break. Also the curable compositions (Comparative Examples 5 and 6) having almost the same compositions as those of curable compositions used in Examples 1 and 2 of the patent document 6 had insufficient pencil hardness and scratch resistance though they had good curability and bending resistance.

### INDUSTRIAL APPLICABILITY

As described above, the cured products obtained by curing the curable compositions of the present invention are preferable as coating materials and optical films for purpose of decoration or protection. For example, they are used in fields where a balance between strength and flexibility is required such as liquid crystal televisions, personal computers, displays of cell phones, touch panels and lenses of watches, and they are useful as optical materials serving as coating materials.

Further, the curable compositions of the present invention can be used as paints providing coating films having an excellent balance among curability, strength and flexibility, and can also be used as adhesives having excellent curability.

## Claims

1. A urethane compound represented by the following general formula (1): 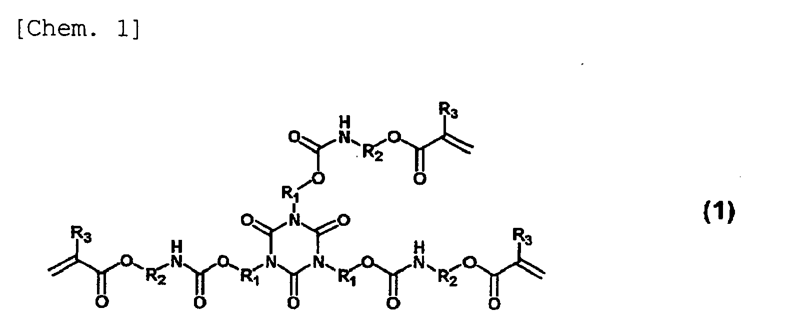 wherein R₁ is a straight-chain or branched divalent aliphatic group of 1 to 12 carbon atoms, a divalent organic group of 3 to 12 carbon atoms having an alicyclic group, a divalent organic group having an aromatic ring or [-(CH₂)ₐ-O- (CH₂)_{b}-]_{c} (a and b are each independently an integer of 1 to 10, and c is an integer of 1 to 5),
R₂ is a straight-chain or branched divalent aliphatic group of 1 to 10 carbon atoms, a divalent organic group of 3 to 10 carbon atoms having an alicyclic group, a divalent organic group having an aromatic ring or [-(CH₂)_{d}-O-(CH₂)ₑ-]_{f} (d and e are each independently an integer of 1 to 10, and f is an integer of 1 to 5), and
R₃ is a hydrogen atom or a methyl group.

2. The urethane compound as claimed in claim 1, wherein in the general formula (1), R₁ is a divalent aliphatic group of 1 to 6 carbon atoms,
R₂ is a divalent saturated aliphatic group of 1 to 6 carbon atoms, a divalent organic group having an benzene ring or [-(CH₂)_{d}-O-(CH₂)ₑ-]_{f} (d and e are each independently an integer of 1 to 5, and f is an integer of 1 to 3), and
R₃ is a hydrogen atom.

3. A curable composition containing the urethane compound (A) as claimed in claim 1 and a polymerization initiator (B).

4. The curable composition as claimed in claim 3, further containing a urethane oligomer (C).

5. The curable composition as claimed in claim 3 or 4, further containing a reactive monomer (D).

6. The curable composition as claimed in any one of claims 3 to 5, further containing a thiol compound (E).

7. The curable composition as claimed in claim 6, wherein the thiol compound (E) has two or more structures represented by the following general formula (2): 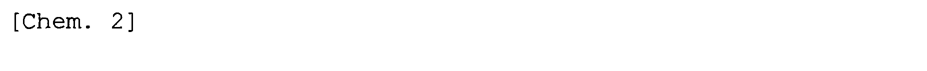 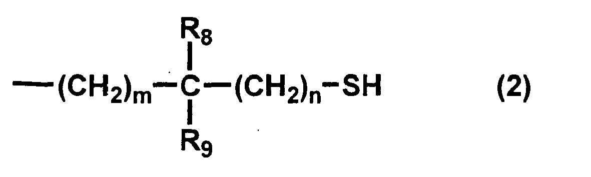 wherein R₈ and R₉ are each independently a hydrogen atom, an alkyl of 1 to 10 carbon atoms or an aryl group, m is an integer of 0 to 2, n is 0 or 1, and R₈ and R₉ are not hydrogen atoms at the same time.

8. The curable composition as claimed in any one of claims 3 to 7, further containing a urethane compound (F) represented by the following general formula (3): 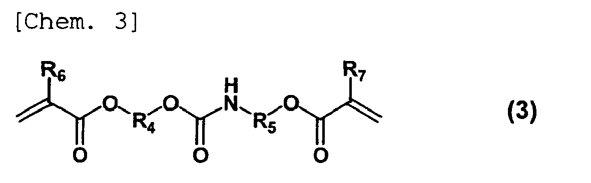 wherein R₄ is a straight-chain or branched divalent aliphatic group of 1 to 12 carbon atoms, a divalent organic group of 3 to 12 carbon atoms having an alicyclic group, a divalent organic group having an aromatic ring or [-(CH₂)_{g}-O-(CH₂)ₕ-]ᵢ (g and h are each independently an integer of 1 to 10, and i is an integer of 1 to 5),
R₅ is a straight-chain or branched divalent aliphatic group of 1 to 10 carbon atoms, a divalent organic group of 3 to 10 carbon atoms having an alicyclic group, a divalent organic group having an aromatic ring or [-(CH₂)ₚ-O-(CH₂)_{q}-]ᵣ (p and q are each independently an integer of 1 to 10, and r is an integer of 1 to 5), and
R₆ and R₇ are each independently a hydrogen atom or a methyl group.

9. A paint comprising the curable composition as claimed in any one of claims 3 to 8.

10. An adhesive comprising the curable composition as claimed in any one of claims 3 to 8.

11. A cured product obtained by curing the curable composition as claimed in any one of claims 3 to 8.

12. A coating material obtained by curing the curable composition as claimed in any one of claims 3 to 8.

13. An optical film obtained by curing the curable composition as claimed in any one of claims 3 to 8.
